Europäisches Patentamt

European Patent Office

Office européen des brevets

(19)

(11) Veröffentlichungsnummer: **0 457 371 A1**

## (12) EUROPÄISCHE PATENTANMELDUNG

(21) Anmeldenummer: 91112543.3

(22) Anmeldetag: **15.12.87**

(51) Int. Cl.⁵: **A61K 35/50**, A61K 37/02

Diese Anmeldung is am 26 - 07 - 1991 als Teilanmeldung zu der unter INID-Kode 60 erwähnten Anmeldung eingereicht worden.

(30) Priorität: **18.12.86 DE 3643182**

(43) Veröffentlichungstag der Anmeldung:
**21.11.91 Patentblatt 91/47**

(60) Veröffentlichungsnummer der früheren Anmeldung nach Art. 76 EPÜ: **0 271 885**

(84) Benannte Vertragsstaaten:
**AT BE CH DE ES FR GB GR IT LI LU NL SE**

(71) Anmelder: **BEHRINGWERKE Aktiengesellschaft Postfach 1140 W-3550 Marburg 1(DE)**

(72) Erfinder: **The designation of the inventor has not yet been filed**

(74) Vertreter: **Klein, Otto, Dr. et al Hoechst AG Zentrale Patentabteilung Postfach 80 03 20 W-6230 Frankfurt am Main 80(DE)**

(54) **Verfahren zur Gewinnung des Gewebeproteins PP4.**

(57) Es wird ein Verfahren zur Gewinnung des Gewebeproteins PP4 an einem hydrophoben, wasserunlöslichen Adsorbens beschrieben.

EP 0 457 371 A1

Die Erfindung betrifft ein Verfahren zur Gewinnung des Gewebeproteins PP4.

In DE 33 15 000 A 1 (USP 4,507,229) ist das Glycoprotein PP4 beschrieben, das beispielsweise in Placenta gefunden wird. PP4 besitzt ein Molekulargewicht von 35 000 ± 5 000 Dalton und einen isoelektrischen Bereich von pH 4.85 ± 0.15.

Blutgerinnungshemmende Proteine lassen sich aufgrund ihrer Wirkungsweise in mehrere Gruppen einteilen. Sowohl die Gruppe der Proteinase-Inhibitoren, wie beispielsweise Antithrombin III, als auch der Proteinasen, beispielsweise aktiviertes Protein C, bringen zum Teil nachteilige Eigenschaften für eine gerinnungshemmende Therapie mit sich, da sie entweder erst auf der Stufe der aktivierten Gerinnungsenzyme wirken oder aber durch eine Proteolyse von Gerinnungsfaktoren diese desaktivieren, sie "verbrauchen".

In DE 35 33 516 A 1, Seiten 6 bis 8, sind blutgerinnungshemmende Proteine beschrieben, für die ein isoelektrischer Bereich von 4.4 - 4.6 angegeben ist. Diese Proteine werden aus der Intimaschicht größerer Gefäße, beispielsweise Aorta oder Nabelschnur, isoliert.

In Acta Obst. Gynaec. Jpn. 36, No. 12, 2583-2592 (1984) ist ebenfalls ein gerinnungshemmendes Protein aus Placenta beschrieben, das ein Molekulargewicht von 45 000 Dalton hat. In DE 33 15 000 A 1 (USP 4,507,229) sind auch die folgenden Eigenschaften von PP4 beschrieben:

eine elektrophoretische Beweglichkeit im Bereich zwischen der von $alpha_1$ und $alpha_2$ Globulinen; ein Sedimentationskoeffizient $s_{20,w}$ von 3,3 ± 0,2 S; ein Extinktionskoeffizient $E_1$ cm (280 nm) von 5,9 ± 0,6; ein Kohlenhydratanteil von 2,4 ± 0,94 % (g/100 g) (Mannose 0,3 ± 0,2 %, Galactose 0,4 ± 0,2 %, Xylose 0,1 % ± 0,04 %, Glukose 0,2 ± 0,1 %, Glukosamin 1,0 + 2 %, Neuraminsäure 0,4 ± 0,2 %) und die folgenden Aminosäurenzusammensetzung:

| Aminosäure | Reste pro 100 Reste (mol %) | Variations- koeffizient |
|---|---|---|
| Lysin | 6,95 | 1,14 |
| Histidin | 0,97 | 17,4 |
| Arginin | 5,44 | 1,77 |
| Asparaginsäure | 11,41 | 1,68 |
| Threonin | 6,78 | 2,40 |
| Serin | 6,21 | 2,26 |
| Glutaminsäure | 12,25 | 0,43 |
| Prolin | 1,96 | 6,20 |
| Glycin | 6,68 | 3,83 |
| Alanin | 7,92 | 1,67 |
| Cystin 1/2 | 0,77 | 19,5 |
| Valin | 5,34 | 3,80 |
| Methionin | 1,98 | 6,00 |
| Isoleucin | 5,21 | 2,23 |
| Leucin | 11,50 | 0,45 |
| Tyrosin | 3,55 | 4,21 |
| Phenylalanin | 4,07 | 3,77 |
| Tryptophan | 0,93 | 23,9 |

Zur Anwendung als Arzneimittel wird PP4 in größeren Mengen gebraucht. Deshalb ist ein einfaches Gewinnungsverfahren aus Geweben wünschenswert.

Es wurde überraschenderweise gefunden, daß PP4 aus einem Gewebeextrakt oder einer Lösung an eine hydrophobe Trägermatrix gebunden und durch eine anschließende Stufen- oder Gradientenelution weitestgehend von Verunreinigungen befreit werden kann.

Gegenstand der Erfindung ist deshalb ein Verfahren zur Reinigung von PP4, dadurch gekennzeichnet, daß eine PP4 enthaltende Lösung, vorzugsweise ein PP4 enthaltender Gewebeextrakt mit einem hydrophoben, wasserunlöslichen Adsorbens in Kontakt gebracht, das Adsorbens von der überstehenden Lösung abgetrennt, gegebenenfalls mit einem Puffer gewaschen und das PP4 eluiert wird.

Vor oder nach diesem Verfahren können noch gegebenenfalls andere bekannte Reinigungsverfahren wie Ionenaustauschchromatographie, Gelfiltration oder Adsorption an Calciumphosphat oder Hydroxylapatit durchgeführt werden.

In einer bevorzugten Verfahrensweise zur Reinigung von PP4 wird die PP4 enthaltende Lösung bei einem pH-Wert von 5.5 - 9.5, einer Temperatur von 0°C bis 40°C und nach Zusatz von Salzen, beispielsweise nach Zusatz von 50 - 250 g Ammoniumsulfat/l Lösung, Magnesiumsulfat oder 0.5 - 3 mol/l NaCl, LiCl oder KCl, mit einem wasserunlöslichen hydrophoben Adsorbens, beispielsweise einem Trägermaterial, das aliphatische, aromatische oder araliphatische Gruppen trägt, in Kontakt gebracht. Als Trägermaterial können beispielsweise RSepharose, Agarose oder RFractogel verwendet werden. Als aliphatische Gruppen werden vorzugsweise Alkylgruppen mit 1 bis 10 C-Atomen, beispielsweise der Butyl- oder Octyl-Rest, und weiterhin beispielsweise gegebenenfalls substituierte Phenyl- oder Phenylalanylreste oder Benzyl- oder substituierte Benzylreste verwendet. Das Adsorbens wird anschließend von der Lösung abgetrennt, gegebenenfalls mit einer Lösung, die beispielsweise 50 - 250 g/l Ammoniumsulfat oder 0.5 - 3 mol/l NaCl, LiCl oder KCl enthält, gewaschen, und das PP4 mit einer Flüssigkeit geringerer Ionenstärke, gegebenenfalls mittels eines Gradienten, eluiert.

In einer besonders bevorzugten Ausführungsform wird die PP4 enthaltende Lösung bei einem pH-Wert von 6 - 8 nach Zusatz von 144 - 243 g Ammoniumsulfat pro l Lösung mit beispielsweise Phenyl- oder Phenylalanyl-RSepharose oder Agarose, ganz besonders bevorzugt aber bei pH 6.5 - 7.8 nach Zusatz von 170 - 200 g Ammoniumsulfat pro l Lösung mit Phenyl-RSepharose in Kontakt gebracht. Anschließend wird das Adsorbens von der Lösung abgetrennt, beispielsweise mit einer Lösung, die 176 - 196 g Ammoniumsulfat/l Lösung enthält, gewaschen und das PP4 mittels einer Lösung, die 114 - 144 g Ammoniumsulfat/l Lösung enthält, eluiert.

Auch ein gentechnisch hergestelltes PP4 kann im erfindungsgemäßen Sinn gereinigt werden.

Ein nach dem beschriebenen Herstellungsverfahren erhaltenes Präparat zeichnet sich besonders dadurch aus, daß PP4 weitestgehend von Verunreinigungen befreit und die blutgerinnungshemmende Wirkung weitestgehend vorhanden ist.

Die Erfindung soll durch das nachstehende Beispiel erläutert werden.


Beispiel 1

Gewinnung von PP4 aus Plazentagewebe

2 kg tiefgefrorene Plazenta wurden in einem Gewebezerkleinerer homogenisiert. Das Homogenisat wurde 3 mal mit je 1 Liter physiologischer NaCl-Lösung gewaschen. Das Plazentagewebe wurde durch Zentrifugation von der überstehenden Lösung abgetrennt. Das gewaschene Plazentahomogenisat wurde in 3 Liter RTriton X 100-haltigem Puffer A (20 mmol/l Tris HCl, 50 mmol/l NaCl, 2 g/100 ml RTriton X 100 pH 7.4) suspendiert und über Nacht gerührt. Der Extrakt wurde durch Zentrifugation abgetrennt und das Gewebe nochmals mit 2 Liter Puffer A über Nacht extrahiert. Die beiden Extrakte wurden vereinigt (4.8 Liter) und mit Puffer B (20 mmol/l Tris/HCl, 50 mmol/l NaCl pH 7.5) auf 15 l verdünnt. Nach Zusatz von 0,75 g DEAE-RSephadex A 50/l Lösung wurde die Suspension 1 Stunde gerührt, das Adsorbat von der Lösung abgetrennt, mit 40 Liter Puffer B, danach mit 600 ml Puffer B, der 100 mmol/l NaCl enthielt, gewaschen und PP4 mit Puffer B, der 500 mmol/l NaCl enthielt, eluiert. Das Eluat (600 ml) wurde mit 176 g/l Ammoniumsulfat versetzt und entweder im Batch-Verfahren oder auf einer Säule mit 300 ml Phenyl-RSepharose behandelt.

Die Phenyl-RSepharose war in Puffer B, der 3/10 der Sättigungskonzentration Ammoniumsulfat enthielt, äquilibriert. Nach Waschen mit demselben Puffer wurde PP4 mit Puffer B, der 1/5 der Sättigungskonzentration Ammoniumsulfat enthielt, eluiert. Das PP4 wurde durch Erhöhen der Ammoniumsulfat-Konzentration auf 85 g/100 ml ausgefällt oder mittels Ultrafiltration konzentriert und über eine Gelfiltration an ACA 54 weitergereinigt. Die ACA 54 Säule (3 x 100 cm) war in Puffer B, der 500 mmol/l NaCl enthält, äquilibriert. Die PP4 enthaltenden Fraktionen wurden vereint.

Bei Bedarf kann sich noch eine Pasteurisierung anschließen. Die Erhitzung kann aber auch auf einer anderen Stufe, wie des DEAE-RSephadex A50-Eluates oder des Eluates der Phenyl-RSepharose-Behandlung durchgeführt werden.

3

**Patentansprüche**

1. Verfahren zur Gewinnung von PP4, dadurch gekennzeichnet, daß eine PP4 enthaltende Lösung, vorzugsweise ein PP4 enthaltender Gewebeextrakt, unter Zusatz eines Salzes mit einem hydrophoben, wasserunlöslichen Adsorbens in Kontakt gebracht, das Adsorbens von der überstehenden Lösung abgetrennt, gegebenenfalls mit einem Puffer gewaschen und das PP4 eluiert wird.

2. Verfahren nach Anspruch 1, dadurch gekennzeichnet, daß das Adsorbens ein Trägermaterial, das aliphatische oder aromatische Gruppen trägt, ist.

3. Verfahren nach Anspruch 1, dadurch gekennzeichnet, daß das wasserunlösliche hydrophobe Adsorbens aus einem Trägermaterial, das aliphatische, aromatische oder araliphatische Gruppen trägt, besteht.

4. Verfahren nach Anspruch 3, dadurch gekennzeichnet, daß als Trägermaterial [R]Sepharose, Agarose oder [R]Fractogel verwendet wird.

5. Verfahren nach Anspruch 3, dadurch gekennzeichnet, daß als aliphatische, aromatische oder araliphatische Gruppen eine Alkylgruppe mit 1 bis 10 C-Atomen, vorzugsweise der Butyl- oder Octyl-Rest, ein substituierter Phenyl- oder Phenylalanylrest oder ein Benzyl- oder substituierter Benzylrest verwendet werden.

6. Verfahren nach Anspruch 1, dadurch gekennzeichnet, daß ein Salz in einer Konzentration von 50 - 250 g oder 0.5 - 3 mol/l Lösung zugesetzt wird.

7. Verfahren nach Anspruch 1, dadurch gekennzeichnet, daß Ammoniumsulfat, Magnesiumsulfat, NaCl, LiCl oder KCl zugesetzt wird.

8. Verfahren nach Anspruch 1, dadurch gekennzeichnet, daß die PP4 enthaltende Lösung bei einem pH-Wert von 6 - 8 nach Zusatz von 144 - 243 g eines Salzes, vorzugsweise Ammoniumsulfat, pro l mit Phenyl- oder Phenylalanyl-[R]Sepharose oder -Agarose in Kontakt gebracht, das Adsorbens von der Lösung abgetrennt, mit einer Lösung, die 176 - 196 g Salz, vorzugsweise Ammoniumsulfat pro l Lösung enthält, gewaschen und das PP4 mittels einer Lösung, die 114 - 144 g eines Salzes, vorzugsweise Ammoniumsulfat, pro l Lösung enthält, eluiert.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | EP-A-0 123 307   (BEHRINGWERKE)<br>* Seite 4, Zeile 21 - Seite 6, Zeile 8 *<br>- - - | 1-8 | A 61 K 35/50<br>A 61 K 37/02 |
| Y | US-A-4 461 833   (GORDON)<br>* Seite 6, Zeilen 12-26 *<br>- - - | 1-8 | |
| A | EUROPEAN JOURNAL OF BIOCHEMISTRY, Band 151, Nr. 3, September 1985, Seiten 625-629, FEBS; C.P.M. REUTE-LINGSPERGER et al.: "Isolation and partial purification of a novel antocoagulant from arteries of human umbilical cord"<br>- - - | | |
| A | GYNECOLOGY, Band 236, Nr. 4, 1985, Seiten 225-233, Springer-Verlag, Berlin, DE; H. BOHN et al.: "Isolation and characterization of two membrane-associated placental tissue proteins"<br>- - - - - | | |

RECHERCHIERTE
SACHGEBIETE (Int. Cl.5)

A 61 K
C 07 K

Der vorliegende Recherchenbericht wurde für alle Patentansprüche erstellt

| Recherchenort | Abschlußdatum der Recherche | Prüfer |
|---|---|---|
| Den Haag | 05 September 91 | TURMO Y BLANCO C.E. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

 

& : Mitglied der gleichen Patentfamilie, übereinstimmendes Dokument